(19) Europäisches Patentamt
European Patent Office
Office européen des brevets

(11) Veröffentlichungsnummer: **0 111 452**
**A1**

(12) # EUROPÄISCHE PATENTANMELDUNG

(21) Anmeldenummer: 83810569.0

(22) Anmeldetag: 05.12.83

(51) Int. Cl.³: A 01 N 43/36
A 01 N 43/40, A 01 N 47/04
A 01 N 57/24

(30) Priorität: 10.12.82 CH 7213/82

(43) Veröffentlichungstag der Anmeldung:
20.06.84 Patentblatt 84/25

(84) Benannte Vertragsstaaten:
BE DE FR GB IT SE

(71) Anmelder: CIBA-GEIGY AG
Postfach
CH-4002 Basel(CH)

(72) Erfinder: Nyfeler, Robert, Dr.
Bärenfelserstrasse 8
D-4057 Basel(CH)

(72) Erfinder: Lorenz, Joachim, Dr.
Im Tiefen Weg 17
D-6140 Bensheim 3(DE)

(72) Erfinder: Grade, Reinhardt, Dr.
Tulpenweg 11
D-6140 Bensheim(DE)

(54) **Verwendung von Pyrrolen als Biocide für den Materialschutz.**

(57) Pyrrolderivate der Formel

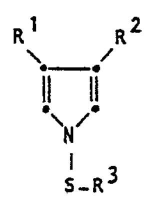

,

worin $R^1$, $R^2$ und $R^3$ die im Anspruch 1 angegebene Bedeutung haben, eignen sich ausgezeichnet für die Verwendung als Biocide im Materialschutz. Besondere Erwähnung gebührt ihr Einsatz im Holzschutz, der Wasserbehandlung, in der Papierindustrie, in Kunststoffen, wie Weich-PVC-Folien, in Dispersionsfarben, Antifouling-Farben und in Bohr- und Schneidölen.

EP 0 111 452 A1

CIBA-GEIGY AG
Basel (Schweiz)

3-14219/+

Verwendung von Pyrrolen als Biocide für den Materialschutz

Die vorliegende Erfindung betrifft die Verwendung N-sulfenylierter Pyrrolderivate als Biocide für den Materialschutz, sowie Materialschutzmittel enthaltend mindestens eines dieser Biocide.

Aus der DE-OS 29 27 480 ist es bekannt, N-Acetylpyrrol-Derivate im Pflanzenschutz einzusetzen.

Die vorliegende Erfindung betrifft N-sulfenylierte Pyrrol-Derivate, welche sich ausgezeichnet für den Materialschutz eignen.

Gegenstand der Erfindung ist somit die Verwendung der Verbindungen der Formel I

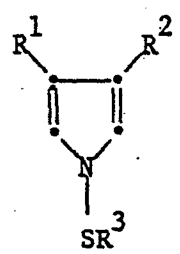

(I) ,

worin $R^1$ unsubstituiertes oder substituiertes Phenyl, Biphenyl, Pyridyl, Furyl oder Thienyl bedeutet, $R^2$ für $-COOR^4$, $-C(O)R^4$, $-CON(R^4)_2$, $-CN$, $-NO_2$, $-SO-R^4$, $-SO_2-R^4$, $-P(O)-(R^5)_2$ oder für $-SO_2-N(R^6)_2$ steht, wobei $R^4$ $C_1$-$C_6$ Alkyl, $R^5$ $C_1$-$C_6$ Alkoxy und $R^6$ $C_1$-$C_3$ Alkyl bedeutet und $R^3$ $C_1$-$C_3$ Halogenalkyl ist, als Biocide für den Materialschutz.

Bevorzugt werden Verbindungen der Formel I verwendet, worin $R^1$ unsubstituiertes oder mit mindestens einem der Reste $R^7$, $R^8$ und/oder

$R^9$ substituiertes Phenyl oder Biphenyl ist und $R^1$ ferner unsubstituiertes oder mit mindestens einem der Reste $R^{10}$, $R^{11}$ und $R^{12}$ substituiertes Pyridyl, Furyl oder Thienyl bedeutet, wobei $R^7$, $R^8$ und $R^9$ unabhängig voneinander für Halogen, $C_1$-$C_4$ Alkyl, $C_1$-$C_3$ Halogenalkyl, $(R^4)_2N$-, $-NO_2$, -CN, $-COOR^4$ oder $-CON(R^4)_2$ steht oder eine der Gruppen $-OR^{13}$, $-SR^{13}$, $S(O)R^{13}$ oder $-SO_2-R^{13}$ ist, wobei $R^{13}$ $C_1$-$C_6$ Alkyl, $C_2$-$C_8$ Alkoxyalkyl, $C_3$-$C_5$ Alkenyl, $C_3$-$C_5$ Halogenalkenyl, $C_3$-$C_5$ Alkienyl, $C_3$-$C_5$ Halogenalkinyl, $C_3$-$C_5$ Hydroxyalkinyl, Phenyl oder Benzyl bedeutet, und $R^{10}$, $R^{11}$ und $R^{12}$ unabhängig voneinander Halogen oder $C_1$-$C_4$ Alkyl bedeuten, und $R^{10}$ zudem $-NO_2$ bedeutet und $R^2$, $R^3$, $R^4$, $R^5$ und $R^6$ die oben angegebene Bedeutung haben.

Der Ausdruck Biphenyl steht für 2-, 3- oder 4-Biphenyl, Pyridyl für 2-, 3- oder 4-Pyridyl, Furyl für 2- oder 3-Furyl und Thienyl für 2- oder 3-Thienyl.

Bevorzugte Reste $R^1$ sind 2- oder 4-Biphenyl, 2- oder 3-Pyridyl, 2-Thienyl und insbesondere Phenyl, 2- oder 3-Pyridyl sowie 2-Furyl. Spezielle Erwähnung gebührt Phenyl.

$R^4$ und $R^6$ sind als Alkyl bevorzugt Methyl, Aethyl, n-Propyl, Isopropyl und $R^4$ ausserdem z.B. n-Butyl, sec.-Butyl, tert.-Butyl, n-Amyl, tert.-Amyl oder n-Hexyl. Besonders bevorzugt ist Aethyl und insbesondere Methyl.

$R^5$ als $C_1$-$C_6$ Alkoxy ist z.B. bevorzugt Methoxy, Aethoxy, Propyloxy und ausserdem Butoxy, Pentyloxy oder Hexyloxy. Besonders bevorzugt ist Methoxy und insbesondere Aethoxy.

Die bevorzugte Bedeutung von $R^2$ ist $-SO_2CH_3$ und insbesondere $-COCH_3$ und -CN.

$R^3$ ist als $C_1$-$C_3$ und bevorzugt $C_1$-$C_2$ Halogenalkyl beispielsweise $-CCl_3$, $-CCl_2F$, $-CCl_2H$, $-CF_3$, $-CF_2H$, $-C_2Cl_5$ oder $-CCl_2CHCl_2$, bevorzugt $-CCl_3$, $-CCl_2F$, $-CCl_2CHCl_2$ und insbesondere $-CCl_2F$.

Ist $R^1$ Phenyl oder Biphenyl, so können diese Reste bevorzugt unsubstituiert sein, oder aber einen oder bis zu drei Substituenten $R^7$, $R^8$ oder $R^9$ tragen.

Ist $R^1$ Pyridyl, Furyl oder Thienyl, so können diese Reste bevorzugt unsubstituiert sein, oder aber einen oder bis zu drei Substituenten $R^{10}$, $R^{11}$ oder $R^{12}$ tragen.

Sind $R^7$, $R^8$, $R^9$, $R^{10}$, $R^{11}$ und $R^{12}$ $C_1$-$C_4$ Alkyl, so handelt es sich beispielsweise um Methyl, Aethyl, n-Propyl, Isopropyl, n-Butyl, sec.-Butyl oder tert.-Butyl. Bevorzugt handelt es sich um Aethyl und insbesondere um Methyl.

$R^7$, $R^8$, $R^9$, $R^{10}$, $R^{11}$ und $R^{12}$ sind als Halogen, Fluor, Chlor, Brom oder auch Jod, bevorzugt handelt es sich um Chlor oder Brom.

Ist $R^7$, $R^8$ oder $R^9$ $C_1$-$C_3$ Halogenalkyl, so kann es sich dabei um einfach bis per-halogenierte Alkylreste handeln, wie $-CH_2Cl$, $-CHCl_2$, $-CCl_3$, $-CH_2Br$, $-CHBr_2$, $CBr_3$, $-CH_2F$, $-CHF_2$, $-CF_3$, $-CCl_2F$, $-CCl_2-CHCl_2$, $-CH_2CH_2F$ oder $CI_3$.

$R^7$, $R^8$ und $R^9$ können ausserdem $-NO_2$, $-CN$, $-SOR^{13}$ oder $-SO_2R^{13}$, sowie bevorzugt $(R^4)_2N-$, $-COOR^4$, $-CON(R^4)_2$, $-OR^{13}$ oder $-SR^{13}$ bedeuten.

In diesen Resten hat $R^4$ die oben beispielhaft erwähnte Bedeutung.

$R^{13}$ ist als $C_1$-$C_6$ und bevorzugt $C_1$-$C_4$ Alkyl z.B. Methyl, Aethyl, n-Propyl, Isopropyl, n-Butyl, sec.-Butyl, tert.-Butyl, n-Amyl, tert.-Amyl oder n-Hexyl.

Als $C_2$-$C_8$ Alkoxyalkyl kann $R^{13}$ z.B. Methoxymethyl, Aethoxymethyl, Methoxyäthyl, Butoxymethyl, Butoxyäthyl oder Hexoxyäthyl bedeuten.

Ist $R^{13}$ $C_3$-$C_5$ Alkenyl, so steht dieser Ausdruck für einen ungesättigten, aliphatischen Rest mit einer oder mit mehreren Doppelbindungen, so z.B. für Propenyl-1, Allyl, Butenyl-1, Butenyl-2 oder $CH_3CH{=}CHCH{=}CH-$, bevorzugt für Allyl. Diese Alkenylreste können mit einem oder mehreren Halogenatomen, wie Fluor, Chlor oder Brom substituiert sein.

$R^{13}$ als Alkinyl ist insbesondere Aethinyl oder Propargyl, welches durch Halogen oder Hydroxy substituiert ist, wie etwa im Rest $-C{\equiv}C-C(OH)(CH_3)_2$.

Bedeutet $R^7$, $R^8$ oder $R^9$ eine Gruppe $-SR^{13}$, so kann $R^{13}$ die angegebene Bedeutung haben. Bevorzugt handelt es sich um eine Gruppe $-SR^{14}$, wobei $R^{14}$ als $C_1$-$C_4$ Alkyl beispielsweise Methyl, Aethyl, n-Propyl, Isopropyl, n-Butyl, sec.-Butyl, Isobutyl oder tert.-Butyl ist.

Bevorzugt werden ausserdem Verbindungen der Formel I verwendet, wobei $R^7$, $R^8$ und $R^9$ Fluor, Chlor, Brom, Methyl, Aethyl, Trifluormethyl, $(R^4)_2N-$, $-COOR^4$, $-CON(R^4)_2$ sind, oder eine Gruppe $-OR^{13}$ oder $-SR^{14}$ bedeuten, und $R^4$ Methyl oder Aethyl ist, und $R^{13}$ $C_1$-$C_4$ Alkyl, $C_3$-$C_5$ Alkenyl, $C_3$-$C_5$ Halogenalkyl, Phenyl oder Benzyl bedeutet und $R^{14}$ $C_1$-$C_4$ Alkyl ist, und $R^{10}$, $R^{11}$ und $R^{12}$ Chlor, Brom, Methyl oder Aethyl sind, und $R^2$ $-COOR^6$, $-C(O)R^6$, $-CON(R^6)_2$, $-CN$, $-NO_2$, $-SO_2R^6$, $-P(O)(R^5)_2$ oder $-SO_2(NR^6)_2$ ist, wobei $R^5$ Methoxy oder Aethoxy ist und $R^6$ Methyl oder Aethyl ist, und $R^3$ $C_1$-$C_2$ Halogenalkyl bedeutet.

Besonders bevorzugt werden Verbindungen der Formel I verwendet, wobei $R^1$ Phenyl, Pyridyl oder Furyl ist, und $R^7$, $R^8$ und $R^9$ Chlor, Brom, Methyl, Aethyl, Trifluormethyl, $-N(CH_3)_2$, $-COOCH_3$, $-CON(CH_3)_2$, $-OR^{13}$ oder $-SR^{14}$ sind, wobei $R^{13}$ $C_1$-$C_4$ Alkyl, Allyl, Phenyl oder

Benzyl ist, und $R^{14}$ $C_1$-$C_4$ Alkyl bedeutet, und $R^{10}$, $R^{11}$ und $R^{12}$ Chlor, Brom oder Methyl ist, $R^2$ die oben angegebene Bedeutung hat, wobei $R^5$ Aethoxy und $R^6$ Methyl ist, und $R^3$ $-CCl_3$, $-CCl_2F$, $-CCl_2H$, $-CF^3$, $-CF_2H$, $-C_2Cl_5$ oder $-CCl_2-CHCl_2$ ist.

Beispiele für Verbindungen der Formel I sind solche, welche der Formel I* entsprechen:

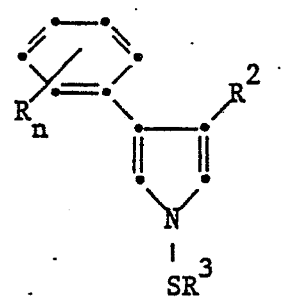

(I*)

| $R_{(n)}$ | $R^2$ | $R^3$ |
|---|---|---|
| 2-n-$OC_3H_7$ | -CN | $-CCl_2F$ |
| 2-n-$OC_4H_9$ | -CN | $-CCl_2F$ |
| 2-n-$SC_3H_7$ | -CN | $-CCl_2F$ |
| 2,3-Dichlor | $-C(O)CH_3$ | $-CCl_2F$ |
| 2,3-Dichlor | $-C(O)CH_3$ | $-CCl_3$ |
| 4-Chlor | $-C(O)CH_3$ | $-CCl_2F$ |
| 2-$SCH_3$ | $-C(O)CH_3$ | $-CCl_2F$ |
| 2-$OCH_3$ | $-C(O)CH_3$ | $-CCl_3$ |
| 4-$N(CH_3)_2$ | $-C(O)CH_3$ | $-CCl_2F$ |
| 3-$CF_3$ | $-C(O)CH_3$ | $-CCl_2F$ |
| 4-Cl | $-CON(CH_3)_2$ | $-CCl_2F$ |
| 4-Cl | $-SO_2CH_3$ | $-CCl_2F$ |
| 4-$OCH_3$ | $-SO_2CH_3$ | $-CCl_3$ |
| 2-n-$SC_4H_9$ | $-SO_2CH_3$ | $-CCl_2F$ |
| 3-Cl | $-SO_2N(CH_3)_2$ | $-CCl_2F$ |

| $R_{(n)}$ | $R^2$ | $R^3$ |
|---|---|---|
| 3-Cl | $-P(O)(OC_2H_5)_2$ | $-CCl_2F$ |
| 4-Cl | $-P(O)(OC_2H_5)_2$ | $-CCl_3$ |
| 2-Cl | $-SOCH_3$ | $-CCl_2F$ |
| 2-Cl | $-SOCH_3$ | $-CCl_3$ |

Weitere Beispiele für Verbindungen der Formel I, worin die Substituenten folgende Bedeutung haben:

| $R^1$ | $R^2$ | $R^3$ |
|---|---|---|
| 2-Furyl | -CN | $-CCl_2F$ |
| 2-Pyridyl | -CN | $-CCl_2F$ |
| 2-Pyridyl | -CN | $-CCl_3$ |
| 2-Pyridyl | $-COOCH_3$ | $-CCl_2F$ |
| 2-Pyridyl | $-COOCH_3$ | $-CCl_3$ |
| 2-Pyridyl | $-CON(CH_3)_2$ | $-CCl_2F$ |
| 2-Pyridyl | $-CON(CH_3)_2$ | $-CCl_3$ |
| 3-(2-Chlorpyridyl) | -CN | $-CCl_2F$ |
| 3-(2-Chlorpyridyl) | -CN | $-CCl_3$ |
| 3-(2-Chlorpyridyl) | $-CO-CH_3$ | $-CCl_2F$ |
| 3-(2-Chlorpyridyl) | $-CO-CH_3$ | $-CCl_3$ |
| 2-(6-Chlorpyridyl) | -CN | $-CCl_2F$ |
| 2-(6-Chlorpyridyl) | -CN | $-CCl_3$ |
| 2-(6-Chlorpyridyl) | $-COOCH_3$ | $-CCl_2F$ |
| 2-(6-Chlorpyridyl) | $-COOCH_3$ | $-CCl_3$ |
| 2-Thienyl | -CN | $-CCl_2F$ |
| 2-Thienyl | -CN | $-CCl_3$ |
| 2-Thienyl | $-CON(CH_3)_2$ | $-CCl_2F$ |
| 2-Thienyl | $-CON(CH_3)_2$ | $-CCl_3$ |

Die Verbindungen der Formel I werden dadurch hergestellt, dass man ein freies Pyrrol der Formel II

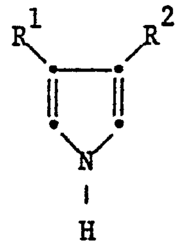

(II)

in Gegenwart einer Base mit einem reaktionsfähigen Säurederivat einer Sulfensäure der Formel III

$$R_3\text{-S-OH} \quad \text{(III)}$$

am Pyrrol-Stickstoff sulfenyliert, wobei die Substituenten $R^1$, $R^2$ und $R^3$ die unter der allgemeinen Formel I angegebenen Bedeutungen haben.

Für diese Sulfenylierungsreaktion eignen sich als reaktionsfähige Sulfensäurederivate z.B. die Niederalkylester und bevorzugt die Sulfensäurehalogenide, insbesondere die -chloride und -bromide und von diesen wiederum besonders die -chloride. Niederalkyl steht hierbei für $C_1$-$C_6$ Alkyl.

Als Basen können sowohl organische als auch anorganische Basen mit Erfolg eingesetzt werden. Geeignete anorganische Basen sind z.B. Alkali- und Erdalkalicarbonate, wie Natriumcarbonat, Kaliumcarbonat, Calciumcarbonat usw. Geeignete organische Basen sind z.B. tertiäre Amine, wie Trialkylamine (Triäthylamin, Methyldiäthylamin), N,N-Dimethoxycyclohexylamin, N-Methylpiperidin, N,N-Dimethylanilin oder Pyridine. Bevorzugt werden die Trialkylamine. Vorteilhafterweise wird die Base in stöchiometrischer Menge oder im Ueberschuss, z.B. bis zu 100% Ueberschuss, in bezug auf das Pyrrol der Formel II eingesetzt. Auch die Verbindung der Formel III wird entweder in stöchiometrischer Menge oder im Ueberschuss zugesetzt.

Die Sulfenylierungs-Reaktion kann in An- oder Abwesenheit, vorzugsweise in Anwesenheit eines reaktionsinerten Lösungsmittels oder Lösungsmittelgemisches, durchgeführt werden. Prinzipiell eignen sich für diese Umsetzung die üblichen organischen Lösungsmittel, sofern sie keine reaktiven Wasserstoffatome enthalten. Geeignet sind beispielsweise aliphatische und aromatische Kohlenwasserstoffe, wie Benzol, Toluol, Xylole, Petroäther, halogenierte Kohlenwasserstoffe, wie Chlorbenzol, Methylenchlorid, Aethylenchlorid, Chloroform, Tetrachlorkohlenstoff, Tetrachloräthylen; Aether und ätherartige Verbindungen, wie Dialkyläther (Diäthyläther, Diisopropyläther, tert.-Butylmethyläther usw.), Aethylenglykol- und Diäthylenglykoldiäther und -monoäther mit je 1-4 C-Atomen in den Alkylteilen, wie Aethylenglykoldimethyl-, -diäthyl- und -di-n-butyläther, Diäthylenglykoldiäthyl- und -di-n-butyläther, Aethylenglykolmonomethyläther und Diäthylenglykolmonomethyläther; Furan, Dimethoxyäthan, Dioxan. Tetrahydrofuran, Anisol; Sulfone, wie Dimethylsulfoxid; Ketone, wie Aceton, Methyläthylketon; Ester, wie Aethylacetat, Propylacetat; Butylacetat und Gemische solcher Lösungsmittel untereinander. In einigen Fällen kann auch das Sulfenylierungsreagenz der Formel III selbst als Lösungsmittel fungieren.

Zur Beschleunigung der Reaktionsgeschwindigkeit kann unter Umständen ein Reaktionskatalysator, wie 4-Dimethylaminopyridin, zugesetzt werden.

Die Sulfenylierungsreaktion wird im allgemeinen bei Temperaturen zwischen -30° bis +100° C, vorzugsweise -10° bis +20° C, durchgeführt. Die Reaktionszeiten liegen dann erfahrungsgemäss etwa zwischen 0,5 h bis 20 h. Durch Zugabe eines Reaktionskatalysators erreicht man u.U. jedoch oft eine Verkürzung der Reaktionsdauer auf weniger als 0,5 h.

Die freien Sulfensäuren der Formel III sind bei norameln Temperaturen im allgemeinen relativ instabile Substanzen, die zur Selbstoxidation neigen. Stabil sind dagegen die an sich bekannten Sulfensäurehalogenide, die z.B. durch Halogenierung aus den zugrundeliegenden Merkaptanen oder Dialkyldisulfiden zugänglich sind. Bekannt sind auch Niederalkylsulfensäureester, die beispielsweise durch Reaktion von Sulfensäurechloriden mit Alkalialkoholaten hergestellt werden können. Sie lagern sich in der Wärme in die entsprechenden Sulfoxide um, sind jedoch bei tieferen Temperaturen handhabbar.

Einige der Verbindungen der Pyrrole der Formel II sind aus der Literatur bekannt. So werden z.B. die Herstellungsmethode und die chemischen Eigenschaften von 4-Cyano-3-phenyl-pyrrol in Tetrahedron-Letters No. 52, S. 5337-5340 (1972) beschrieben.

Unterschiedlich substituierte 3-Phenyl-4-cyanopyrrol-derivate sind aus der Literatur bekannt. So werden beispielsweise Pyrrole der Formel

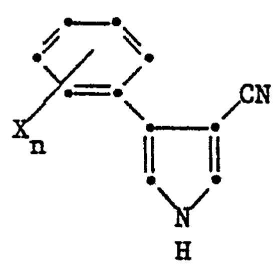

(IV) ,

worin X ein Halogenatom, eine niedere Alkyl- oder niedere Halogenalkylgruppe bedeutet und n für 0, 1 oder 2 steht, in der DE-OS 29 27 480 als Zwischenprodukte mit geringer fungizider Aktivität beschrieben.

Die Verbindungen der Formel II lassen sich in alkalischem Medium durch cyclisierende Michael-Addition einer Verbindung der Formel V mit p-Tolylsulfonylmethylisocyanid unter Abspaltung von p-Toluolsulfinsäure bzw. ihres Salzes herstellen.

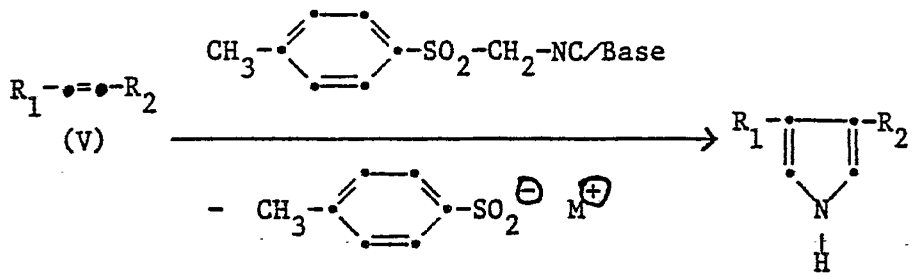

(II) .

Dabei sind die Substituenten $R_1$ und $R_2$ wie oben definiert.

Die p-Tolylsulfonyl-Gruppe steht hier und im folgenden stellvertretend für eine ganze Reihe von Gruppen, die in der Lage sind, die Methylengruppe im Methylisocyanid-Rest für eine Reaktion im Sinne einer Michael-Addition zu aktivieren. Weitere bevorzugte Beispiele für derartig aktivierende Gruppen sind Benzolsulfonyl-, p-Chlorbenzolsulfonyl, Niederalkylsulfonyl, wie Mesyl.

Die Cycloaddition wird vorteilhafterweise in Gegenwart einer nicht nukleophilen Base durchgeführt. Als Basen eignen sich Alkalimetallhydride, wie Natriumhydrid oder Alkali- bzw. Erdalkalicarbonate, wie $Na_2CO_3$, $K_2CO_3$, oder Alkalialkoholate, wie $(CH_3)_3CO^{\ominus}K^{\oplus}$ und andere. Die Base wird vorteilhafterweise in mindestens äquimolarer Menge, bezogen auf die Ausgangsstoffe, eingesetzt.

Wie bei allen Umsetzungen, so ist es auch hier zweckmässig, die Reaktion in einem reaktionsinerten Lösungsmittel durchzuführen. Für die beschriebene Cycloaddition kommen beispielsweise folgende, bevorzugt wasserfreie Lösungsmittel in Frage: Aromatische und aliphatische Kohlenwasserstoffe, wie Benzol, Toluol, Xylole, Petroläther, Ligroin, Cyclohexan; Aether und ätherartige Verbindungen, wie Dialkyläther (Diäthyläther, Diisopropyläther, t-Butylmethyläther usw.), Dimethoxymethan. Dioxan, Tetrahydrofuran, Anisol; Sulfone, wie Dimethylsulfoxid; Dimethylformamid und Gemische solcher Lösungsmittel untereinander.

Die Cycloaddition wird im allgemeinen bei Temperaturen von -30° bis +120° C, bevorzugt bei -30° bis +50° C bzw. am Siedepunkt des Lösungsmittels oder Lösungsmittelgemisches, durchgeführt.

Bei der Wahl geeigneter Basen lässt sich die Cycloaddition vorteilhafterweise auch in wässrigem Medium ausführen. Als Basen eignen sich in diesen Fällen wasserlösliche anorganische und organische Basen, insbesondere Alkalihydroxide, wie LiOH, NaOH oder KOH und Ammoniumbasen, z.B. Tetraalkylammoniumhydroxide, wie $(CH_3)_4NOH$. Die Base wird in mindestens äquimolarer Menge, bezogen auf die Ausgangsstoffe, eingesetzt. Bei der Verwendung wässriger Basen führt man die Reaktion vorteilhafterweise in einem heterogenen Zweiphasensystem durch.

Für die organische, mit Wasser nicht mischbare Phase kommen z.B. folgende Lösungsmittel in Frage: Aliphatische und aromatische Kohlenwasserstoffe, wie Pentan, Hexan, Cyclohexan, Petroläther, Ligroin, Benzol, Toluol, Xylole usw., halogenierte Kohlenwasserstoffe, wie Dichlormethan, Chloroform, Tetrachlorkohlenstoff, Aethylendichlorid, 1,2-Dichloräthan, Tetrachloräthylen usw., oder aliphatische Aether, wie Diäthyläther, Diisopropyläther, t-Butylmethyläther usw.

Zur Beschleunigung der Reaktionsgeschwindigkeit kann bei dieser Durchführungsart die Gegenwart eines Phasentransfer-Katalysators von Vorteil sein. Beispiele derartiger Katalysatoren sind: Tetraalkylammoniumhalogenide, -hydrogensulfate oder -hydroxide, wie Tetrabutylammoniumchlorid, -bromid, -jodid; Triäthylbenzylammoniumchlorid, -bromid; Tetrapropylammoniumchlorid, -bromid, -jodid usw. Als Phasentransfer-Katalysatoren kommen auch Phosphonium-Salze in Betracht.

Die Phasentransfer-katalysierte Cycloaddition kann bei Temperaturen von 0° bis +80° C, vorzugsweise +10° bis +50° C bzw. beim Siedepunkt des Lösungsmittelgemisches, durchgeführt werden.

Die Cycloaddition kann in den beschriebenen Ausführungsarten bei Normaldruck erfolgen; die Reaktionsdauer beträgt im allgemeinen 1 bis 16 Stunden, bei Phasentransferkatalyse 0,5 bis 10 Stunden.

Mit den erfindungsgemässen Verbindungen wird ein breites Wirkungsspektrum in der Bekämpfung von Mikroorganismen und von tierischen und pflanzlichen Schädlingen geschaffen, woraus sich vielerlei Verwendungsmöglichkeiten, z.B. als Bactericide, Desinfektionsmittel, gegen Schleimbildung in der Papierherstellung, als Fungizide, Insektizide, Akarizide, Herbizide oder Algizide ergeben. Sie sind ideale Produkte für die Wasserbehandlung zur Kontrolle von Mikroorganismen, zum Reinigen von Kühlwassersystemen und/oder zur Verhinderung von Schleimbildung.
Ausserdem eignen sich die neuen Stoffe ausgezeichnet als industrielle Antimikrobika zum Materialschutz, z.B. Schützen von Holz, Zellstoff und Papier, Textilien und Leder, von Farben, Lacken, Antifoulingfarben und ähnlichen Beschichtungsstoffen, von optischen und anderen Gläsern, Kunststoffen, Gummi und Klebestoffen, von Bohr- und Schneideölen, von Erdöl, Schmierstoffen, Wachsen und Treibstoffen und anderen Materialien.

Die Wirkung der erfindungsgemäss verwendeten Verbindungen kann auch in konservierenden und desinfizierenden Ausrüstungen von Kunststoffen, z. B. Polyamiden, Polyurethanschäume oder Polyvinylchlorid, ausgenützt werden. Bei der Verwendung von Weichmachern ist es vorteilhaft, den antimikrobiellen Zusatz dem Kunststoff im Weichmacher gelöst bzw. dispergiert zuzusetzen. Die Kunststoffe mit antimikrobieller Ausrüstung können für Gebrauchsgegenstände aller Art, bei denen eine Wirksamkeit gegen verschiedenste Keime, wie Bakterien und Pilze, erwünscht ist, Verwendung finden, so z.B. in Weich-PVC-Folien, für Fussmatten, Badezimmervorhänge, Sitzgelegenheiten, Trittroste in Schwimmbädern, Wandbespannungen etc.

Die Verbindungen werden je nach Verwendungszweck in den dem Fachmann bekannten Konzentrationsbereichen eingesetzt. Die Grenzen der gebräuchlichen Konzentrationen sind durch folgende Werte gegeben: Während in Kühlwasser bereits Konzentrationen im ppm-Bereich genügen, so sind in Antifoulingrezepturen Konzentrationen bis 40 Gew.-% üblich.

Typische Konzentrationen der Biocide im Materialschutz sind: 0,01-10 Gew.-% in Holzschutzmitteln, 1-100 ppm bei der Wasserbehandlung und in der Papierindustrie, 0,1-5 Gew.-% bei der Ausrüstung von Kunststoffen, wie Weich-PVC-Folien, 0,1-40 Gew.-% in Dispersionsfarben und Antifoulingfarben, sowie 0,01-1 Gew.-% in Bohr- und Schneideölen.

Die Verbindungen können in reiner Form oder zusammen mit Trägerstoffen als Stäube-, Streu- oder Nebelmittel appliziert werden. Sie können auch in flüssigen Streichmitteln u.ä. suspendiert werden, wobei gegebenenfalls zur Bildung von gleichmässigen Dispersionen, Netzmittel oder Emulgiermittel die gleichmässige Verteilung des Wirkstoffs fördern kann. Es können weitere Biocide, wie Insektizide, hinzugefügt werden.

Uebliche Grundstoffe für Antifoulingfarben sind die als Bindemittel bezeichneten und dem Fachmann bekannten Lackrohstoffe, wie natürliche und synthetische Harze, homo- und copolymere Produkte mit den Monomeren Vinylchlorid, Vinylidenchlorid, Styrol, Vinyltoluol, Vinylestern, Acrylsäuren und Methacrylsäure, sowie deren Estern, ferner Chlorkautschuk, natürlicher und synthetischer Kautschuk, gegebenenfalls chloriert oder cyclisiert, auch Reaktionsharze, wie Epoxidharze, Polyurethanharze, ungesättigte Polyester, die gegebenenfalls durch Zusatz von Härtern in filmbildende, höhermolekulare Produkte übergeführt werden können.

Die Bindemittel können flüssig sein oder in gelöster Form vorliegen. Bei gelösten Bindemitteln, auch Thermoplasten, kann ein Schutzfilm auch durch Verdampfen des Lösungsmittels gebildet werden. Feste Beschichtungsmittel können z. B. im Pulverbeschichtungsverfahren auf Gegenstände aufgebracht werden. Weitere übliche Grundstoffe sind z. B. Teer, Modifikatoren, Farbstoffe, anorganische oder organische Pigmente, Füllstoffe und Härter.

Die Erfindung umfaßt somit ferner Mittel, die die erfindungsgemäßen Verbindungen enthalten, sowie die Verwendung der erfindungsgemäßen Verbindungen und Mittel zur Bekämpfung von Mikroorganismen und Algen im Materialschutz.

Die erfindungsgemäß verwendbaren Biocidgemische können auch weitere Aktivsubstanzen enthalten.

Beispiele dafür sind:

a) Organo-Schwefelverbindungen, z. B. Methylendithiocyanat, Isothiazolone, Benzisothiazolone, 3,5-Dimethyltetrahydro-1,3,5-2H-thiodiazin-2-thio (DMTT).

b) Phenole, z. B. Natriumpentachlorphenolat, Chlorkresole.

c) Stickstoffverbindungen, z. b. s-Triazine, quarternäre Ammoniumverbindungen, N-Dichlorfluoromethylthio-N-Aryl-Sulfamide.

d) Halogenverbindungen, z. B. Dibromcyanoacetamid, 1.2-Dibrom-2.4-dicyanobutan, Polychlorophthalonitrile, Halogenacetamide-

e) Kupfersalze, wie Kupfersulfat.

f) Bei der Wasserbehandlung sind außerdem Chlor und Brom, Chlordioxid, Chlorisocyanuarate und Hypochlorite gängige Biocide.

g) Bei der Holzbehandlung Salzgemische auf Basis Silicofluoride, Hydrogenfluoride, anorganische Borverbindungen, Chromate, Fluoride, Arsen (Oxid, Arsenate), Kupfersalze (Sulfat, Naphthenat), Zinn- und Zinksalze, Quecksilberverbindungen, auch Teerölpräparate sowie organische Wirkstoffe, wie Pentachlorphenol, Phenol, DDT, Dieldrin, Lindan, Gammexan, chlorierte Naphthaline, Organozinnverbindungen.

h) In Desinfektionsmitteln und Konservierungsmitteln Phenol oder Phenolderivate, Formaldehyd und/oder sonstige Aldehyde bzw. deren Derivate, Chlor, organische oder anorganische Substanzen mit aktivem Chlor, Amphotenside, Alkohole.

i) In Ölen und Treibstoffen z. B. Borverbindungen.

Selbstverständlich können in solchen Formulierungen außerdem noch weitere Substanzen und Hilfsmittel enthalten sein, wie sie üblicherweise in solchen Zubereitungen mitverwendet werden. Hierzu gehören z. B. ionische oder nichtionische oberflächenaktive Substanzen.

Elektrolyte, Komplexbildner, Lösungsvermittler sowie Farb- und Duftstoffe. Diese Zusätze dienen beispielsweise zur Verbesserung des Netzvermögens, der Härtungsstabilität, zur Viskositätseinstellung und zur Erhöhung der Kältestabilität der Lösungen.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid. Druckangaben erfolgen in Millibar mb, oder Bar b. THF steht für Tetrahydrofuran.

## Herstellungsbeispiele

α) Ausgangsprodukte

Beispiel a: Herstellung von

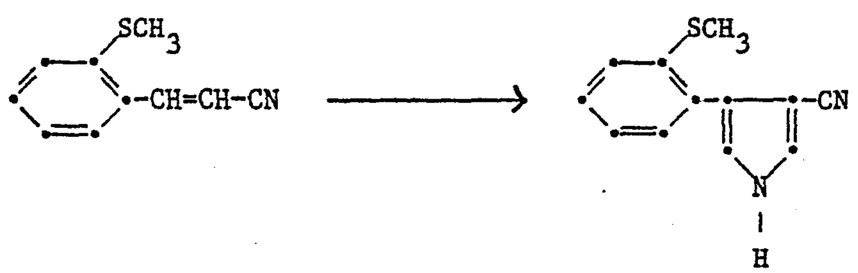 (Nr. 24)

3-(2-Methylthiophenyl)-4-cyanopyrrol

Zu einer Lösung von 158 g (1,4 Mol) Kalium-tert.-butylat in 400 ml THF lässt man bei -13° bis +8° langsam eine Lösung von 123 g (0,7 Mol) E/Z-3-(2-Thiomethylphenyl)-propennitril und 192 g (0,98 Mol) p-Toluolsulfonylmethylisocyanid in 800 ml THF zutropfen. Anschliessend wird das Gemisch noch 2,25 Stunden bei RT gerührt, dann auf 3 Liter Eiswasser gegossen und zweimal mit Essigsäureäthylester extrahiert. Die vereinigten Extrakte werden viermal mit halbgesättigter Natriumchloridlösung gewaschen, über Natriumsulfat getrocknet, mittels Kieselgel und Aktivkohle entfärbt, filtriert und eingeengt. Der Rückstand wird durch Zugabe von Dichlormethan/Petroläther zur Kristallisation gebracht. Ausbeute 95 g beigefarbener Kristalle. Fp. 121-124°. Durch Aufarbeitung der Mutterlauge werden weitere 21 g der Substanz erhalten. Durch mehrfache Umkristallisation aus Dichlormethan/Petroläther erhält man ein fast farbloses Produkt mit Fp. 128-134°.

Beispiel b: Herstellung von

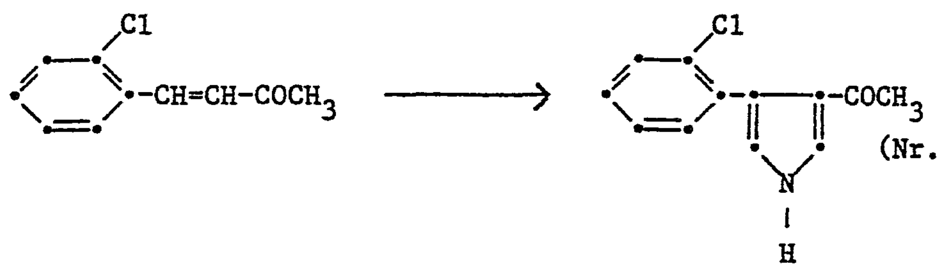

(Nr. 49)

3-(2-Chlorphenyl)-4-acetylpyrrol

Zu 48 g (0,43 Mol) Kalium-tert.-butylat in 100 ml THF lässt man bei -10° bis 30° langsam eine Lösung von 60 g (0,33 Mol) E/Z-4-(2-Chlorphenyl)-3-buten-2-on und 75 g (0,38 Mol) p-Toluolsulfonylmethylisocyanid in 400 ml THF zutropfen. Anschliessend wird das Gemisch noch 2,5 Stunden bei 5° bis 10° gerührt, dann auf RT erwärmt, auf 2 Liter Eiswasser gegossen und zweimal mit Essigsäureäthylester extrahiert. Die vereinigten Extrakte werden viermal mit halbgesättigter Natriumchloridlösung gewaschen, über Natriumsulfat getrocknet, mit Kieselgel und Bleicherde entfärbt, filtriert und eingeengt. Der Rückstand wird in Dichlormethan unter Rückfluss digeriert, abgekühlt und filtriert. Ausbeute 40 g beigefarbener Kristalle. Fp. 198-201°.

Beispiel c: Herstellung von

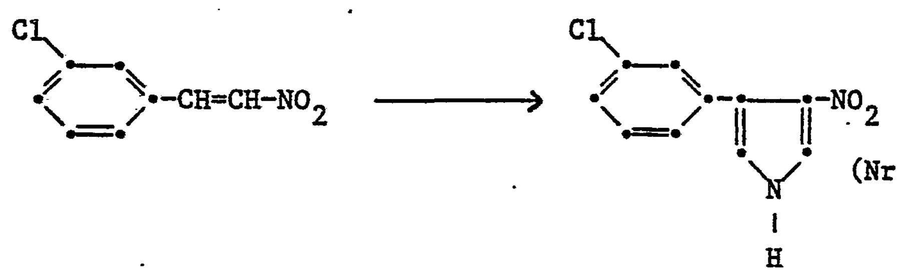

(Nr. 41)

3-(3-Chlorphenyl)-4-nitropyrrol

1,6 g (0,036 Mol) einer ca. 55%igen Natriumhydrid-Dispersion in Mineralöl werden unter Stickstoffatmosphäre zweimal mit Petroläther digeriert und dann mit 50 ml Diäthyläther versetzt. Zu dieser Mischung lässt man unter kräftigem Rühren eine Lösung von 5,5 g (0,03 Mol) 2-[3-(Chlorphenyl)-1-nitroäthen und 5,9 g (0,03 Mol) p-Toluolsulfonylmethylisocyanid in 20 ml DMSO und 40 ml Diäthyläther so

zutropfen, dass das Reaktionsgemisch ständig unter Rückfluss siedet. Nach Abklingen der exothermen Reaktion wird noch 15 Minuten bei RT weitergerührt. Anschliessend wird das Gemisch zuerst vorsichtig mit Eiswasser und dann mit gesättigter Natriumchloridlösung versetzt und zweimal mit Essigsäureäthylester extrahiert. Die vereinigten Extrakte werden viermal mit halbgesättigter Natriumchloridlösung gewaschen, über Natriumsulfat getrocknet, filtriert und eingeengt. Der Rückstand wird aus Dichlormethan/Petroläther umkristallisiert und führt zu gelben Kristallen mit Fp. 133-135°.

Beispiel d: Herstellung von

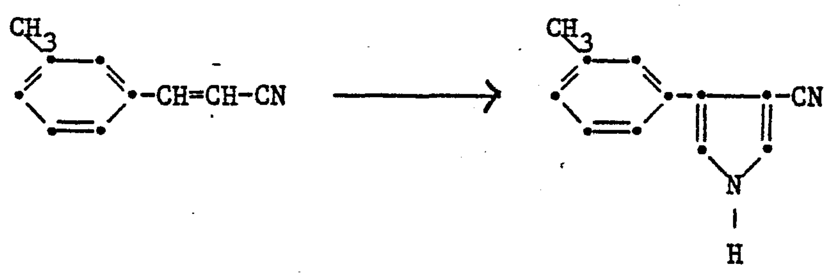

(Nr. 10)

3-(3-Methylphenyl)-4-cyanopyrrol

6,1 g Natriumhydrid-Dispersion (55%-ig in Mineralöl) werden mit Petroläther gewaschen und mit 60 ml Dimethoxyäthan und 10 ml DMSO versetzt. Unter Rühren lässt man dazu bei -25° bis -20° langsam eine Lösung von 14,3 g 3-Methylzimtsäurenitril und 21,5 g p-Toluolsulfonylmethylisocyanid in 60 ml Dimethoxyäthan und 10 ml DMSO zutropfen, rührt noch 1,5 Stunden bei auftauendem Eisbad, giesst das Gemisch auf Eis und extrahiert zweimal mit Essigsäureäthylester. Die organische Phase wird mit wässriger Natriumchloridlösung gewaschen, über Natriumsulfat getrocknet, filtriert und eingeengt. Es werden 19 g eines viskosen Oels erhalten, das nach Umkristallisation aus Dichlormethan/Petroleumbenzin kristallines 3-(3-Methylphenyl)-4-cyanopyrrol liefert. Fp. 109-111°.

Beispiel e: Herstellung von

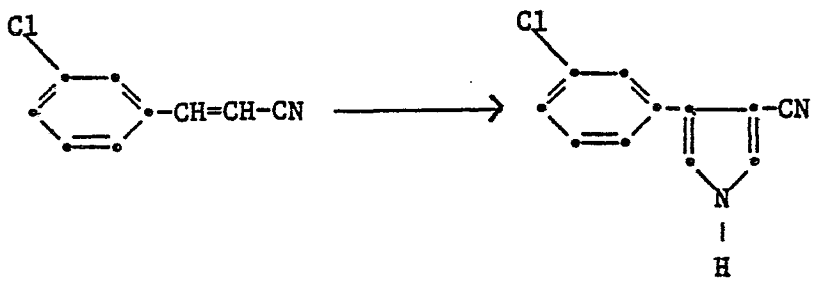 (Nr. 1)

3-(3-Chlorphenyl)-4-cyanopyrrol

a) 10,5 g Natriumhydrid-Dispersion (55%ig in Mineralöl) werden mit Petroläther gewaschen und anschliessend mit 120 ml THF versetzt. Danach lässt man bei -25° bis -20° eine Lösung aus 30,7 g 3-Chlorzimtsäurenitril und 41 g p-Toluolsulfonylmethylisocyanid in 180 ml THF und 20 ml DMSO zutropfen und rührt das Gemisch noch 2 Stunden bei aufgetautem Eisbad. Anschliessend wird die Mischung auf Eis gegossen, zweimal mit Essigsäureäthylester extrahiert, die organische Phase mehrmals mit wässriger Natriumchloridlösung gewaschen, über Natriumsulfat getrocknet, filtriert und das Filtrat eingeengt. Umkristallisation des festen Rückstandes aus Dichlormethan liefert kristallines 3-(3-Chlorphenyl)-4-cyanopyrrol, Fp. 138-140°.

Auf analoge Weise lassen sich alle Zwischenprodukte herstellen. So z.B. jene der Formel

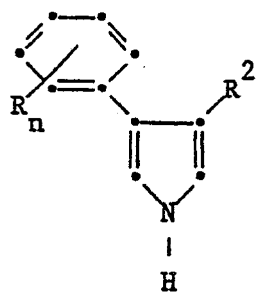

| Verb. Nr. | $R_{(n)}$ | $R^2$ | Fp. [°C] |
|---|---|---|---|
| 1 | 3-Cl | CN | 138-140° |
| 2 | 2,4-$Cl_2$ | CN | 150-152° |
| 3 | 4-Cl | CN | 153-155° |
| 4 | 2-Cl | CN | 136-138° |
| 5 | 4-F | CN | 137-138° |
| 6 | 3-F | CN | 138-139° |
| 7 | 3-Br | CN | 132-134° |
| 8 | 4-$N(CH_3)_2$ | CN | 180-182° |
| 9 | 3-$N(CH_3)_2$ | CN | 144-146° |
| 10 | 3-$CH_3$ | CN | 109-111° |
| 11 | 3-$NO_2$ | CN | 232-234° |
| 12 | 3-$CF_3$ | CN | 87-89° |

| Verb.Nr. | $R_{(n)}$ | $R^2$ | Fp. [°C] |
|---|---|---|---|
| 13 | $3\text{-}OC_6H_5$ | CN | 124-126° |
| 14 | $3\text{-}OCH_3$ | CN | 125-128° |
| 15 | $3\text{-}OCHF_2$ | CN | 95-97° |
| 16 | $2\text{-}OCH_3$ | CN | 135-136° |
| 17 | $2\text{-}OCHF_2$ | CN | 105-107° |
| 18 | $2\text{-}OC_2H_5$ | CN | 134-135° |
| 19 | $2\text{-}OCH(CH_3)_2$ | CN | 80-81° |
| 20 | $2\text{-}OCH_2CH{=}CH_2$ | CN | 83-86° |
| 21 | $2\text{-}OCH_2C_6H_5$ | CN | 91-93° |
| 22 | 2-OH | CN | 130-132° |
| 23 | $3\text{-}SCH_3$ | CN | 115-117° |
| 24 | $2\text{-}SCH_3$ | CN | 128-134° |
| 25 | $2\text{-}SO\text{-}CH_3$ | CN | 168-171° |
| 26 | $2\text{-}SO_2\text{-}CH_3$ | CN | 150-152° |
| 27 | $2\text{-}SC_2H_5$ | CN | 121-123° |
| 28 | $2\text{-}SO\text{-}C_2H_5$ | CN | 144-145° |
| 29 | $2\text{-}SO_2\text{-}C_2H_5$ | CN | 141-143° |
| 30 | $2\text{-}S\text{-}CH(CH_3)_2$ | CN | 121-123° |
| 31 | $2\text{-}SO_2\text{-}CH(CH_3)_2$ | CN | 139-141° |
| 32 | $2\text{-}S\text{-}C_4H_9\text{-}n$ | CN | 61-65° |
| 33 | H | CN | |
| 34 | $2\text{-}SO_2C_4H_9\text{-}n$ | CN | 151-152° |
| 35 | $2\text{-}COOC_2H_5$ | CN | 130-132° |
| 36 | $3\text{-}C{\equiv}C\text{-}C(OH)(CH_3)_2$ | CN | Oel |
| 37 | $3\text{-}C{\equiv}C\text{-}H$ | CN | 132-134° |
| 38 | $2\text{-}C{\equiv}C\text{-}C(OH)(CH_3)_2$ | CN | 140-143° |
| 39 | $2\text{-}C{\equiv}C\text{-}H$ | CN | 109-115° |
| 40 | 3-Cl | $CO\text{-}OCH_3$ | 187-189° |
| 41 | 3-Cl | $NO_2$ | 133-135° |
| 42 | 2-Cl | $NO_2$ | 137-139° |

| Verb. Nr. | $R_{(n)}$ | $R^2$ | Fp. [°C] |
|---|---|---|---|
| 43 | 3-Cl | $P(O)(OC_2H_5)_2$ | 102-104° |
| 44 | 3-Cl | $P(O)(OCH_3)_2$ | 120-122° |
| 45 | 2-Cl | $P(O)(OC_2H_5)_2$ | 129-131° |
| 46 | 2-Cl | $P(O)(OCH_3)_2$ | 146-148° |
| 47 | $2,4-Cl_2$ | $P(O)(OC_2H_5)_2$ | 89-90° |
| 48 | 3-Cl | $CO-CH_3$ | 193-195° |
| 49 | 2-Cl | $CO-CH_3$ | 198-201° |
| 50 | $2,4-Cl_2$ | $CO-CH_3$ | 184-186° |
| 51 | H | $CO-CH_3$ | 156-158° |
| 52 | $3-CH_3$ | $CO-CH_3$ | 120-123° |
| 53 | 4-Cl | $CO-CH_3$ | 170-174° |
| 54 | $3,4-Cl_2$ | $CO-CH_3$ | 162-164° |
| 55 | $4-OCH_3$ | $CO-CH_3$ | 179-182° |
| 56 | $4-CH_3$ | $CO-CH_3$ | 181-184° |
| 57 | 3-Cl | $SO_2-CH_3$ | 125-127° |
| 58 | 4-Cl | $SO_2-CH_3$ | 154-156° |
| 59 | $2,4-Cl_2$ | $SO_2-CH_3$ | 174-177° |
| 60 | $3-NO_2$ | $SO_2-CH_3$ | 133-135° |
| 61 | 2-Cl, $4-NO_2$ | $SO_2-CH_3$ | 197-200° |
| 62 | 2-Cl | $SO_2-CH_3$ | 178-180° |

und auch jene der Formel

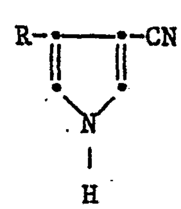

| Verb. Nr. | R | Fp. [°C] |
|---|---|---|
| 63 | 2-Pyridyl | 148-151° |
| 64 | 3-Pyridyl | 160-162° |
| 65 | 4-Pyridyl | 200-203° |
| 66 | 2-(6-Chlorpyridyl) | 204-206 |

| Verb. Nr. | R | Fp. [°C] |
|---|---|---|
| 67 | 3-(2-Chlorpyridyl) | 217-220° |
| 68 | 2-(3,5-Dichlorpyridyl) | 176-179° |
| 69 | 2-Thienyl | 122-124° |
| 70 | 2-Furyl | 98-100° |
| 71 | 2-(N-Methylpyrrol) | 174-175° |
| 72 | 2-(6-Methylthienyl) | 108-110° |
| 73 | 2-(3-Methylthienyl) | 132-137° |

β) Endprodukte

Beispiel 1: Herstellung von

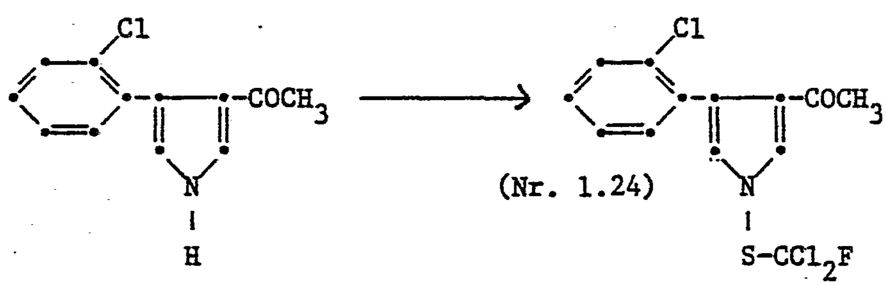

(Nr. 1.24)

N-Fluordichlormethylsulfenyl-3-(2-chlorphenyl)-4-acetylpyrrol

Zu einer Lösung von 11,0 g (0,05 Mol) 3-(2-Chlorphenyl)-4-acetylpyrrol in 100 ml THF lässt man zuerst bei 3° bis 5° eine Lösung von 6 ml (0,0575 Mol) Fluordichlormethylsulfenylchlorid in 20 ml THF und anschliessend bei 5° bis 8° 8 ml (0,0575 Mol) Träthylamin in 20 ml THF zutropfen. Das Gemisch wird dann 16 Stunden bei auftauendem Eisbad gerührt, filtriert und das Filtrat eingeengt. Der Rückstand wird aus Diäthyläther/Petroläther umkristallisiert. Ausbeute 9,4g beiger Kristalle mit Fp. 85-87°. Die chromatographische Reinigung (Kieselgel, Dichlormethan/Petroläther 4:1) und Einengung der Mutterlauge liefert nach Umkristallisation aus Diäthyläther/Petroläther weitere 4,5 g beiger Kristalle mit Fp. 84-86°.

Beispiel 2: Herstellung von

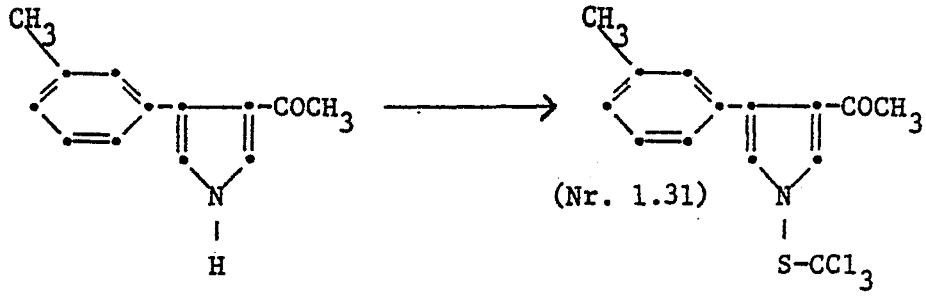

(Nr. 1.31)

N-Trichlormethylsulfenyl-3-(3-methylphenyl)-4-acetylpyrrol

Zu einer Lösung von 6 g (0,03 Mol) 3-(3-Methylphenyl)-4-acetylpyrrol in 100 ml THF lässt man zuerst bei 0° bis 5° 3,0 ml (0,036 Mol) Trichlormethylmercaptan in 30 ml THF und anschliessend bei 5° bis 8° 5 ml (0,036 Mol) Triäthylamin in 30 ml THF zutropfen. Das Reaktionsgemisch wird dann 16 Stunden bei auftauendem Eisbad gerührt, filtriert und das Filtrat eingeengt. Der Rückstand wird säulenchromatographisch (Kieselgel, Dichlormethan/Petroläther 2:1) gereinigt und gibt nach Umkristallisation aus Petroläther 5 g farbloser Kristalle mit Fp. 58-59°.

Beispiel 3a: Herstellung von

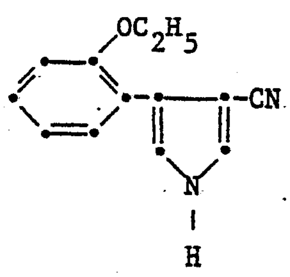

(Nr. 1.15)

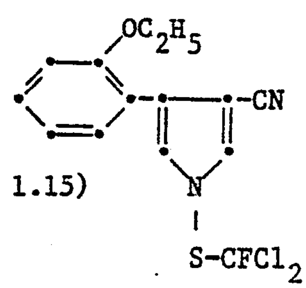

N-Fluordichlormethylsulfenyl-3-(2-äthoxyphenyl)-4-cyanopyrrol

Zu einer Lösung von 7,2 g (0,034 Mol) 3-(2-Aethoxyphenyl)-4-cyanpyrrol in 100 ml Essigsäureäthy lester lässt man zuerst bei 3° bis 7° eine Lösung von 4 ml (0,039 Mol) Fluordichlormethylsulfenylchlorid in 15 ml Essigsäureäthylester und anschliessend 5,4 ml (0,039 Mol) Triäthylamin in 15 ml Essigsäureäthylester zutropfen und rührt das

Reaktionsgemisch noch 16 Stunden bei auftauendem Eisbad. Dann wird filtriert und das Filtrat eingeengt. Der Rückstand wird aus Petroläther umkristallisiert. Ausbeute: 9,9 g beiger Kristalle mit Fp. 80-83°.

Beispiel 3b:

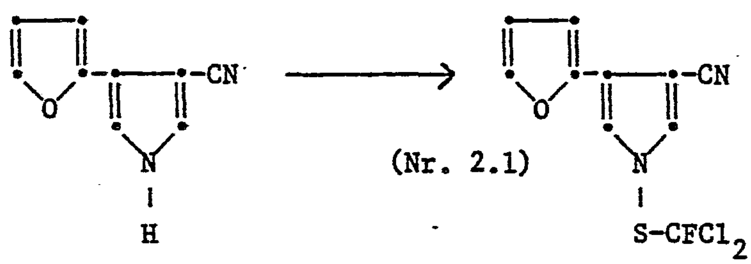

N-Fluordichlormethylsulfenyl-3-(2-furyl)-4-cyanopyrrol

Zu einer Lösung von 5,2 g (0,033 Mol) 3-(2-Furyl)-4-cyanopyrrol in 100 ml THF lässt man zuerst bei 0° bis +5° 3,9 ml (0,037 Mol) Fluordichlormethylsulfenylchlorid in 10 ml THF und anschliessend bei 5° bis 8° 5,2 ml (0,037 Mol) Triäthylamin in 10 ml THF zutropfen. Das Reaktionsgemisch wird dann 16 Stunden bei auftauendem Eisbad gerührt, filtriert und das Filtrat eingeengt. Der Rückstand wird säulenchromatographisch (Kieselgel/Dichlormethan/Petroläther 3:1) gereinigt und liefert nach der Umkristallisation aus Petroläther 7 g gelbliche Kristalle mit Fp. 74-76°.

Auf analoge Weise lassen sich auch die übrigen, im folgenden einzeln aufgezählten Verbindungen der Formel I herstellen:

Tabelle: Verbindungen der Formel

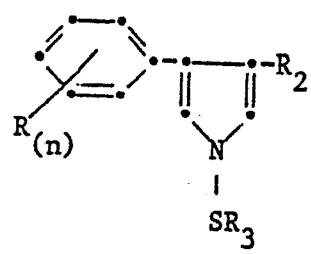

| Verb. Nr. | $R_n$ | $R^2$ | $R^3$ | Fp. [°C] |
|---|---|---|---|---|
| 1.1 | $3-OCH_3$ | -CN | $-CCl_2F$ | Oel |
| 1.2 | $2-S-C_2H_5$ | -CN | $-CCl_2F$ | 59-60° |
| 1.3 | $2-S-C_2H_5$ | -CN | $-CCl_3$ | 90-92° |
| 1.4 | $2-S-CH(CH_3)_2$ | -CN | $-CCl_2F$ | 64-66° |
| 1.5 | $2-S-CH(CH_3)_2$ | -CN | $-CCl_3$ | 100-102° |
| 1.6 | $2-SCH_3$ | -CN | $-CCl_2F$ | 86-87° |
| 1.7 | $2-SCH_3$ | -CN | $-CCl_3$ | 125-127° |
| 1.8 | $2-SC_4H_9-n$ | -CN | $-CCl_2F$ | 44-46° |
| 1.9 | $2-SC_4H_9-n$ | -CN | $-CCl_3$ | 41-42° |
| 1.10 | $2-OCH(CH_3)_2$ | -CN | $-CCl_2F$ | 95-98° |
| 1.11 | $2-OCH(CH_3)_2$ | -CN | $-CCl_3$ | 100-102° |
| 1.12 | 2-Benzyloxy | -CN | $-CCl_2F$ | 127-129° |
| 1.13 | 2-Benzyloxy | -CN | $-CCl_3$ | 111-113° |
| 1.14 | 2-Br | -CN | $-CCl_2F$ | 69-71° |
| 1.15 | $2-OC_2H_5$ | -CN | $-CCl_2F$ | 80-83° |
| 1.16 | $2-OC_2H_5$ | -CN | $-CCl_3$ | 107-110° |
| 1.17 | $2-OCH_2CH=CH_2$ | -CN | $-CCl_2F$ | 66-68° |
| 1.18 | $2-OCH_2CH=CH_2$ | -CN | $-CCl_3$ | 79-80° |
| 1.19 | 3-Cl | -CN | $-CCl_2F$ | 87-89° |
| 1.20 | 3-Cl | -CN | $-CCl_3$ | 81-84° |
| 1.21 | 2-Cl | -CN | $-CCl_2F$ | 59-61° |
| 1.22 | H | $-CO-CH_3$ | $-CCl_2F$ | 43-45° |
| 1.23 | H | $-CO-CH_3$ | $-CCl_3$ | 86-88° |
| 1.24 | 2-Cl | $-CO-CH_3$ | $-CCl_2F$ | 86-88° |
| 1.25 | 2-Cl | $-CO-CH_3$ | $-CCl_3$ | 104-109° |
| 1.26 | $4-CH_3$ | $-CO-CH_3$ | $-CCl_2F$ | 82-84° |

| Verb. Nr. | $R_n$ | $R^2$ | $R^3$ | Fp. [°C] |
|---|---|---|---|---|
| 1.27 | $4-CH_3$ | $-CO-CH_3$ | $-CCl_3$ | 100-102° |
| 1.28 | 3-Cl | $-CO-CH_3$ | $-CCl_2F$ | 63-64° |
| 1.29 | 3-Cl | $-CO-CH_3$ | $-CCl_3$ | 82-84° |
| 1.30 | $3-CH_3$ | $-CO-CH_3$ | $-CCl_2F$ | $n_D^{52}$: 1.5871 |
| 1.31 | $3-CH_3$ | $-CO-CH_3$ | $-CCl_3$ | 58-59° |
| 1.32 | $4-OCH_3$ | $-CO-CH_3$ | $-CCl_2F$ | $n_D^{52}$: 1.5927 |
| 1.33 | $4-OCH_3$ | $-CO-CH_3$ | $-CCl_3$ | $n_D^{52}$: 1.6098 |
| 1.34 | 3-Cl | $-COOCH_3$ | $-CCl_2F$ | 77-79° |
| 1.35 | 3-Cl | $-COOCH_3$ | $-CCl_3$ | 89-90° |
| 1.36 | 3-Cl | $-SO_2CH_3$ | $-CCl_2F$ | Oel |
| 1.37 | 3-Cl | $-SO_2CH_3$ | $-CCl_3$ | 104-107° |
| 1.38 | $2,4-Cl_2$ | $-SO_2CH_3$ | $-CCl_2F$ | 106-108° |
| 1.39 | $2,4-Cl_2$ | $-SO_2CH_3$ | $-CCl_3$ | 111-113° |
| 1.40 | 2-Cl | $-SO_2CH_3$ | $-CCl_2F$ | 86-88° |
| 1.41 | $2-OC_2H_5$ | -CN | $-CCl_2CHCl_2$ | 103° Zers. |
| 1.42 | 2-Cl | -CN | $-CCl_3$ | 93-95° |
| 1.43 | $2-OCH_3$ | -CN | $-CCl_2F$ | Harz |
| 1.44 | $2-OCH_3$ | -CN | $-CCl_3$ | Harz |
| 1.45 | 2-Cl | $-SO_2CH_3$ | $-CCl_3$ | 118-121° |
| 1.46 | 2-Cl | $-NO_2$ | $-CCl_2F$ | Harz |
| 1.47 | 2-Cl | $-C(O)CH_3$ | $-CCl_2F$ | |
| 1.48 | H | $-CON(CH_3)_2$ | $-CCl_2F$ | Oel |
| 1.49 | H | -CN | $-CCl_2F$ | 96-98° |
| 1.50 | H | -CN | $-CCl_3$ | 102-103° |
| 1.51 | H | $-SO_2CH_3$ | $-CFCl_2$ | 106-107° |
| 1.52 | 2-Cl | $-COOCH_3$ | $-CFCl_2$ | Oel |
| 1.53 | $2,3-Cl_2$ | -CN | $-CFCl_2$ | 105-107° |
| 1.54 | 2-Cl | $-\overset{O}{\overset{\|}{P}}-(OCH_2CH_3)_2$ | $-CFCl_2$ | Oel |
| 1.55 | 2-Cl | -CN | $-CCl_2-CHCl_2$ | 123-125° |
| 1.56 | Diphenyl | -CN | $-CCl_3$ | 168-175° |

Analog wurden hergestellt ($R^1$, $R^2$ und $R^3$ gemäss Formel I):

| Verb. Nr. | $R^1$ | $R^2$ | $R^3$ | Fp [°C] |
|---|---|---|---|---|
| 2.1 | 2-Furyl | -CN | $-CCl_2F$ | 74 - 76° |
| 2.2 | 2-Pyridyl | -CN | $-CCl_2F$ | 92 - 100° |
| 2.3 | 2-Pyridyl | -CN | $-CCl_3$ | 131 - 140° |
| 2.4 | 4-Pyridyl | -CN | $-CCl_2F$ | ab 145° Zers. |
| 2.5 | 3-Pyridyl | -CN | $-CCl_2F$ | 123 - 126° |
| 2.6 | 3-Pyridyl | -CN | $-CCl_3$ | ab 156° Zers. |
| 2.7 | 4-Pyridyl | -CN | $-CCl_3$ | ab 154° Zers. |

Beispiel 4: Bestimmung der minimalen Hemmkonzentration gegen Bakterien

Die in Caso-Pepton-Bouillon (Merck) gewachsenen üNK's (über Nacht bebrütete Kulturen) der verschiedenen Bakterienstämme:

A) Proteus vulgaris, B) Pseudomonas aeruginosa, C) Enterobacter aerogenes, D) Serratia marcencens, E) Alcaligenes denitrificans, F) Bacillus substilis, werden jeweils in Saline 1/1000 verdünnt. Von den Suspensionen wird soviel in Caso-Pepton-Bouillon gegeben, dass die Bakterien erneut 1/1000 verdünnt werden. Danach werden jeweils in Tabelle angeführte Verbindungen in Mengen von 100 und 300 mg/l zugegeben. Nach einer Bebrütung von 24 Stunden bei 30° C im Schüttelwasserbad wird nach Trübung ausgewertet. Die minimale Hemmkonzentration (MIC) ist die Konzentration, bei der die Bouillon nicht durch Bakterienwuchs trüb wird.

Das Ergebnis ist in der folgenden Tabelle 1 veranschaulicht:

Tabelle 1 Bestimmung der MIC gegen Bakterien (mg/l)
(bei Subst. Nr. 1.22, 1.40, 2.1, 2.2 wurden ausser 100 und 300 mg/l auch die Konzentrationen 10, 30 und 60 mg/l getestet)

| Subst. Nr. | Stamm A | B | C | D | E | F |
|---|---|---|---|---|---|---|
| 1.43 | 100 | 100 | 300 | 100 | 100 | 100 |
| 1.51 | 30 | 100 | 100 | 60 | 30 | 30 |
| 1.6 | 100 | 300 | 100 | 100 | 100 | 100 |
| 1.22 | 10 | 30 | 30 | 30 | 10 | 10 |
| 1.28 | 100 | 300 | >300 | 100 | 100 | 100 |
| 1.40 | 30 | 60 | 60 | 60 | 30 | 30 |
| 1.47 | 100 | 300 | >300 | 300 | 100 | 100 |
| 2.1 | 10 | 30 | 30 | 30 | 10 | 10 |
| 2.2 | 30 | 100 | 60 | 60 | 60 | 30 |
| 2.4 | 10 | 60 | 30 | 30 | 30 | 10 |
| 2.5 | 10 | 30 | 30 | 30 | 10 | 10 |
| 1.37 | 100 | 300 | >300 | >300 | 100 | 100 |
| 1.46 | 100 | 300 | 300 | 100 | 100 | 100 |
| 1.48 | 100 | 100 | >300 | >300 | 100 | 100 |
| 2.3 | 100 | 100 | >300 | >300 | 100 | 100 |

Aus Tabelle 1 ist die gute wuchshemmende Wirkung der Verbindungen vor allem auch gegen die schwierig zu bekämpfenden gram⁻ Bakterien zu erkennen.

Beispiel 5: Bestimmung der minimalen Abtötungskonzentration gegen eine Bakterien-Mischkultur

Von den in Caso-Pepton-Bouillon gewachsenen ÜNK's der verschiedenen Bakterienstämme

G) Escherichia coli

H) Bacillus cereus var. mycoides

I) Staphylococcus aureus

B) Pseudomonas aeruginosa

C) Enterobacter aerogenes

A) Proteus vulgaris

werden zur Herstellung der Mischkultur jeweils soviel in Tyrode, die die Verbindungen mit 10, 30, 60 oder 100 mg/l enthält, gegeben, dass

eine Endverdünnung von 1/1000 erreicht wird, und die Mischkultur 5 Stunden bei 30° C im Schüttelwasserbad bebrütet.

Danach werden aus den Proben 5 ul entnommen und auf Caso-Pepton-Agar getropft. Nach einer erneuten Bebrütung bei 30° C für 24 Stunden wird visuell nach Wuchs ausgewertet.

Wie aus folgender Tabelle 2 zu erkennen, sind ein Teil der Verbindungen gegen diese schleimbildenden Bakterien hoch bakterizid wirksam.

Tabelle 2: Abtötung einer Bakterienmischkultur in Tyrode

| Nr. | Wuchs, Konzentration (mg/l) | | | |
|---|---|---|---|---|
| | 10 | 30 | 60 | 100 |
| 1.19 | + | + | + | - |
| 1.22 | + | + | + | - |
| 1.36 | + | + | + | - |
| 1.51 | + | + | (+) | - |
| 1.2 | (+) | - | - | - |
| 2.1 | (+) | (-) | - | - |
| 2.2 | + | (+) | - | - |
| 2.4 | + | + | - | - |
| 1.40 | + | + | (+) | - |
| 2.5 | + | + | + | - |

\+ = Wuchs, keine Abtötung
(+) = Wuchs weniger als Kontrolle (10 Kolonien), geringe Abtötung
(-) = geringer Wuchs (1 - 10 Kolonien)
\- = kein Wuchs, Abtötung

Die gute abtötende Wirkung macht diese Verbindungen als Wirkstoffe in Desinfektionsmitteln nutzbar.

Beispiel 6: Bestimmung der minimalen Hemmkonzentration gegen Pilze

Die Untersuchung wird mit dem bekannten Agar-Inkorporationstest mit den Pilzen

K) Aspergillus niger
L) Aspergillus phoenicis
M) Penicillium funiculosum
N) Alternaria alternata

O) Cladosporium cladosporioides

P) Candida albicans

Q) Endomyces geotrichum

R) Aureobasidium pullulans

S) Chaetomium globosum

im Malzextrakt-Agar (Merck) durchgeführt. Zur Hemmung werden jeweils soviel an den verschiedenen Verbindungen zugegeben, dass Konzentrationen von 10, 50 und 100 mg/l im Agar resultieren. Die zur Hemmung des Wuchses der Pilze (von aufgetropften Pilzsporen ausgehend) benötigten Konzentrationen (mg/l) sind in Tabelle 3 veranschaulicht.

Tabelle 3 Bestimmung der MIC gegen Pilze (Konz. mg/l)

| | Stamm | | | | | | | | |
|---|---|---|---|---|---|---|---|---|---|
| Nr. | K | L | M | N | O | P | Q | R | S |
| 1.2 | 10 | 50 | 10 | 10 | 10 | 10 | 10 | 10 | 10 |
| 1.6 | 10 | 10 | 10 | 10 | | 10 | 10 | | 10 |
| 1.22 | 10 | 10 | 10 | 10 | | 10 | 10 | | 10 |
| 1.24 | 50 | 50 | 50 | 50 | | 50 | 100 | | 50 |
| 1.26 | 10 | 10 | 10 | 10 | | 10 | 10 | | 10 |
| 1.28 | 10 | 10 | 10 | 10 | | 10 | 10 | | 10 |
| 1.37 | 10 | 10 | 50 | 50 | | 10 | 10 | | 10 |
| 1.40 | 10 | 50 | 10 | 10 | 10 | 50 | 50 | 10 | 10 |
| 1.47 | 10 | 10 | 10 | 10 | | 10 | 50 | | 10 |
| 2.1 | 10 | 10 | 10 | 10 | 10 | 10 | 10 | 10 | 10 |
| 2.2 | 50 | 50 | 50 | 50 | 50 | 50 | 50 | 50 | 50 |
| 2.3 | 10 | 10 | 50 | 10 | 10 | 10 | 10 | | 10 |
| 1.49 | 10 | 10 | 10 | 10 | 10 | 10 | 10 | | 10 |
| 1.50 | 10 | 50 | 10 | 10 | 10 | 10 | 50 | | 10 |

Aus Tabelle 3 ist zu erkennen, dass die Verbindungen hervorragende Fungizide sind. Die Ergebnisse der Beispiele 4, 5 und 6 belegen die Brauchbarkeit dieser Verbindungen zur Konservierung einschließlich ihrer Anwendung in Kosmetika.

Beispiel 7: Bestimmung der Wirkung gegen Algen

a) Chlorella vulgaris

Die in Algen-Nährmedien über 14 Tage gewachsene Kultur von Chlorella vulgaris wird in Algen-Nährmedium 1/200 verdünnt. Danach werden jeweils die in Tabelle 4 angeführten Verbindungen so zugegeben, dass eine Konzentration von 3 mg/l resultiert. Nach 6 Stun-

den Inkubation werden jeweils 10 µl entnommen und zur Bestimmung der abtötenden Wirkung auf Algen-Agar aufgetropft. Nach einer Bebrütung von 14 Tagen unter Belichtung (14 Stunden Licht-, 10 Stunden Dunkel-Wechsel) wird visuell der Wuchs im Algenmedium (Bestimmung der Wuchshemmung) und auf dem Algen-Agar (Bestimmung der algenabtötenden Wirkung) beurteilt.

Tabelle 4 Wirkung gegen Chlorella vulgaris

| Nr. | Wuchs im Medium | Wuchs auf dem Agar |
|---|---|---|
| 1.6 | - | (+) |
| 1.21 | - | - |
| 1.26 | - | - |
| 2.5 | - | - |
| 2.4 | - | - |
| 1.14 | - | - |
| 1.19 | - | (-) |

- = kein Wuchs, auf dem Agar, 3 ppm haben in 6 Stunden die Algen abgetötet

Aus der Tabelle ist die hervorragende Wirkung der Verbindungen gegen die für den Materialschutz so wichtige Alge Chlorella vulgaris zu erkennen.

Beispiel 8: Untersuchung der Wirksamkeit im Kühlturm

Der im Labor unter Beleuchtung (14 h Licht-, 10 h Dunkel-Wechsel) befindliche Kreislauf besitzt folgende technische Daten:

a) Volumen: 27 l
b) Verdunstung: 320 ml/h
c) Abschlemmverlust: 0,8 l/h
d) Frischwasser = Verdunstung + Abschlemmverlust
e) Kühlleistung: $\Delta T = 2°$ C von 26° C auf 24° C

In dem Kühlkreislauf, der mit Leitungswasser gespeist wird, hat sich eine natürliche Bakterienflora entwickelt.

Diese wird mit 10 mg/l, Beispiel 2.1, Beispiel 1.2 oder 30 mg/l Beispiel 1.40 (5% Formulierung in Methylcellusolve behandelt). Ausgewertet wird der Versuch durch Bestimmung des Keimtiters mittels Verdünnen und Ausspateln jeweils direkt vor und nach verschiedenen Zeiten der Zugabe des Biocids.

Tabelle 5

| Stunden nach Dosierung | Keimzahl/ml Nr. 1.49 30 mg/l | Nr. 2.1 10 mg/l | Nr. 1.2 10 mg/l | Nr. 1.40 30 mg/l |
|---|---|---|---|---|
| 0 | $4,4x10^5$ | $1,0x10^5$ | $1,0x10^6$ | $8,4x10^4$ |
| 3 | $9,2x10^3$ | $1,7x10^2$ | $6,7x10^5$ | $2,9x10^3$ |
| 24 | $5,3x10^2$ | $1,3x10^3$ | $7,9x10^5$ | $8,5x10^2$ |
| 48 | $4,8x10^2$ | $7,7x10^5$ | $1,6x10^6$ | $4,5x10^3$ |

Wie aus Tabelle 5 zu erkennen, gelingt es mit Beispiel Nr. 2.1, 1.49 und Nr. 1.40, 99% der Keime in dem Kühlwasser zu töten, Beispiel Nr. 1.2 ist mit 10 mg/l nicht so aktiv, zeigt aber mit dieser niedrigen Konzentration noch einen deutlichen abtötenden Effekt.

Beispiel 9: Untersuchung der Wirksamkeit in Bohr- und Schneidölen

Die Untersuchung findet mit den beiden folgenden Kühlschmierstoffen statt:

a) Konventionelles Bohröl, bestehend aus Mineralöl, Korrosionsschutz- und Stabilisierungszusätzen, sowie anionenaktiven Emulgatoren.
b) Teilsynthetisches Bohröl mit einem Oelgehalt von weniger als 60% und u.a. Hochdruckzusätzen und nicht-ionogene Emulgatoren.

Die beiden Schmierstoffe (Konzentrate mit $H_2O$ 1/20 verdünnt) werden mit einer Bakterien-Hefen-Mischkultur angeimpft (Endverdünnung 1/100), das Biocid mit der Konzentration von 300 bzw. 1500 ppm zugegeben und die Röhrchen bei Zimmertemperatur in der Rollapparatur bebrütet. Nach einer weiteren Woche Inkubation wird erneut beimpft. Vor dem Animpfen werden jeweils Proben entnommen und in Saline 1/1000 verdünnt. Nachdem jeweils 5 µl (2x) auf Caso-Agar-Platten aufgetropft sind und die Platten 3 Tage bei 30° C bebrütet worden sind, wird nach Wuchs ausgewertet. Die zum Animpfen dienende Mischkultur besteht aus folgenden Stämmen (1 : 1 : 1 etc. Verhältnis der üNK's):

Proteus vulgaris
Pseudomonas aeruginosa
Pseudomonas oleovorans
Candida albicans
Endomyces geotrichum
Escherichia coli
Bacillus subtilis
Staphylococcus aureus
Enterobacter aerogenes

Die Verbindungen Nr. 2.1 und 1.40 halten die Bohröle mit 300 mg/l für 2-3 Wochen, mit 1500 mg/l für mehr als 4 Wochen steril.

Beispiel 10: Filtertest

Der Filtertest wird als Vortest für den Holzschutz, um die Wirkung der Produkte gegen Pilze auf Cellulose zu untersuchen, verwendet.

0,1 ml einer Sporensuspension einer Mischkultur (1 + 1 + 1 etc.) von

Aspergillus niger
Aspergillus phoenicis
Penicillium funiculosum
Alternaria alternata
Cladosporium cladosporioides und
Chaetomium globosum

werden auf Malzextrakt-Agar ausgepatelt. Nach 1 h Eintrocknungszeit werden Cellulose-Filterplättchen (Ø 10 mm) auf den Agar gelegt und jeweils 5 µl einer Stammlösung in Methylcellusolve von den erfindungsgemässen Verbindungen mit 20, 100 und 200 mg/l langsam auf das Filterpapier gegeben. Nach 2wöchiger Bebrütung bei 28° C wird nach folgendem Schema ausgewertet:

1 = Wuchs auf dem Filterpapier, wie Kontrolle
2 = Wuchs auf dem Filterpapier, schwächer als Kontrolle
3 = kein Wuchs auf dem Filterpapier
4 = kein Wuchs auf dem Filterpapier, Hemmzone bis 2 mm
5 = kein Wuchs auf dem Filterpapier, Hemmzone  2 mm

Tabelle 6 Wirkung im Filtertest

| Subst. Nr. | 20 mg/l | 100 mg/l | 200 mg/l |
|---|---|---|---|
| 1.6 | 2 | 2-3 | 3 |
| 1.22 | 2 | 3-4 | 3-4 |
| 1.28 | 1 | 3 | 3-4 |
| 1.37 | 1-2 | 2 | 2-3 |
| 1.26 | 2 | 4 | 4-5 |
| 1.47 | 2 | 2-3 | 3-4 |
| 2.2 | 1 | 1 | 2-3 |
| Methylcellusolve 5 µl | 1 *) | | |

Aus der Tabelle ist zu erkennen, dass die Verbindungen auch auf Cellulose hervorragende Fungizide sind und daher für den Holzschutz geeignet sind.    *) Kontrolle

Beispiel 11: Untersuchung der Wirksamkeit in Dispersionsfarben

In je 30 g Dispersionsfarbe (nicht ausgerüstete Aussenfarbe) werden jeweils so viel Biocid mit dem abgeflammten Spatel eingerührt, dass eine Menge von 1% an Biocid erreicht wird.

Die Biocid enthaltende Dispersionsfarbe wird mit einem Spatel auf Filterpapier ($589^2$ Weissbrand Ø 5,5 cm) aufgetragen und anschliessend 3 Tage getrocknet.

Nach dem Trocknen werden die Filterpapiere 5 Tage unter fliessendem Wasser ausgelaugt, 1 Tag getrocknet und danach für jeweils 30 Min. beidseitig mit einer UV-Lampe (Lampe zur Raumdesinfektion, ca. 1 m Entfernung) zur Reduktion der Fremdkeime bestrahlt.

Die Filterpapiere werden dann in der Mitte getrennt, eine Hälfte auf Algen-Agar und die andere Hälfte auf Kartoffel-Glucose-Agar gelegt.

Auf dem Algen-Agar und das Filterpapier werden 0,1 ml einer Algenmischkultur (1 + 1) von
Chlorella vulgaris
Scenedesmus spec. (14tägige Kulturen 1/100 verdünnt mit Saline)
ausgespalt, die Platten bei Raumtemperatur (14 h Licht - 10 h Dunkel-Wechsel) für mindestens 14 Tage inkubiert und nach Wuchs ausgewertet:

1 = Algenbewuchs auf der gesamten Oberfläche des Filterpapiers, wie Kontrolle
2 = Algenbewuchs weniger als Kontrolle
3 = Algenbewuchs nicht deutlich erkennbar
4 = kein Algenbewuchs, Hemmzone = 5 mm
5 = kein Algenbewuchs, Hemmzone 5 mm

Auf dem Kartoffel-Glucose-Agar und die Filterpapierhälfte werden 0,1 ml einer Sporensuspension, einer Pilzmischkultur (1 + 1 + 1) von Aspergillus niger, Aspergillus phenicis, Penicillium funiculosum, Alternaria alternata, Cladosporium cladosporioides, Aureobasidium pullulans und Chaetomium globosum verteilt, 14 Tage bei 28° C inkubiert und nach Wuchs, wie bei den Algen beschrieben, ausgewertet.

Tabelle 7 Wirkung in Dispersionsfarben

| | Wirkung gegen | |
|---|---|---|
| Nr. | Pilze | Algen |
| 1.22 | 3-4 | 4-5 |
| 1.40 | 2 | 4 |

Wie aus der Tabelle zu erkennen, sind die mit den erfindungsgemässen Produkten ausgerüsteten Dispersionsfarben gegen Bewuchs geschützt.

Beispiel 12: Wirkung als Slimicide in Papierwasser

Das zur Zeit der Entnahme unbehandelte Papierwasser einer Papierfabrik war mit einer Bakterien-Keimzahl von 2,7 x $10^6$ K/ml und mit einer Pilzkeimzahl (vor allem Hefen) von $10^8$ - $10^9$ K/ml infiziert.

Zur Bestimmung der abtötenden Wirkung der Verbindungen in diesem Papierwasser werden jeweils 5; 7,5; 10; 15 und 20 mg/l zugegeben, die Proben 3 h behandelt und die Keimzahl durch Auftropfen von 5 µl auf Agar und Bebrütung bestimmt.

Tabelle 8 Abtötende Wirkung gegen in Papierwasser enthaltene Keime

| | Wuchs Konzentration (mg/l) | | | | |
|---|---|---|---|---|---|
| Nr. | 5 | 7,5 | 10 | 15 | 20 |
| 2.1 | (+) | - | - | - | - |
| 1.2 | (+) | (-) | (-) | - | - |
| 1.26 | + | (+) | (+) | (-) | (-) |

+ = Wuchs, keine Abtötung
(+) = Wuchs weniger als Kontrolle ( 10 Kolonien) geringe Abtötung
(-) = geringer Wuchs (1-10 Kolonien)
- = kein Wuchs, Abtötung

Aus der Tabelle ist die hervorragende Wirkung der Verbindungen als Slimicide in Papierwasser zu erkennen.

Beispiel 13: Wirkung in Weich-PVC

In 39,8 g PVC-Paste, bestehend aus 23 g Pasten PVC Konz. (K-Wert 70), 16,1 g Di-2-Aethylhexylphthalat (DOP), 0,46 g epoxidiertes Sojabohnenöl und 0,46 g flüssiger Barium-Cadmium-Stabilisator, werden jeweils 0,08 g oder 0,48 g Biocid eingerührt (abgerieben), auf eine Glasplatte mit einer Schichtdicke von 0,5 mm aufgestrichen und nach 5 Min. Gelieren ein Stück abgezogen.

Zur biologischen Prüfung werden 1 $cm^2$ PVC -Stücke auf Malz-Extrakt-Agar gelegt, auf dem vorher 0,1 ml einer Sporensuspension von jeweils

17 Aspergillus niger
18 Aspergillus phoenicis
14 Penicillium funiculosum
15 Alternaria alternata
22 Cadida albicans
23 Endomyces geotrichum
28 Chaetomium globosum

ausgepatelt ist.

Nach einer Bebrütung von 7 Tagen bei 28° C wird nach folgender Skala ausgewertet:

1 = Bewuchs am Rand der Folie, wie Kontrolle
2 = Bewuchs schwächer als Kontrolle
3 = kein Bewuchs der Folie, Hemmhof 0-2 mm
4 = kein Bewuchs der Folie, Hemmhof 2 mm

| Nr. | Konzentration (g) | Stamm 17 | 18 | 14 | 15 | 22 | 23 | 28 |
|---|---|---|---|---|---|---|---|---|
| 1.22 | 0,08 | 2-3 | 3 | 2 | 2 | 3 | 2 | 3 |
| 1.22 | 0,48 | 3-4 | 3-4 | 3-4 | 2-3 | 4 | 3 | 4 |
| 2.1 | 0,08 | 2 | 2-3 | 2 | 1-2 | 3 | 2 | 2 |
| 2.1 | 0,48 | 3 | 3 | 3-4 | 2 | 3-4 | 3 | 3-4 |

Wie aus der Tabelle zu erkennen, sind die Weich-PVC Folien durch die Ausrüstung mit den Produkten bestens gegen den Bewuchs von Pilzen geschützt.

Beispiel 14: Holzschutztest

Der Holzschutztest wurde in Anlehnung an DIN 52176 mit Splintholz der Kiefer (Abmessung: 50 x 25 x 15 mm) und dem Prüfpilz Coniophora puteana durchgeführt.

Die Probehölzer wurden nach dem Trocknen im Exikator über Kieselgel 15 min einem Vakuum ausgesetzt und dann in Biocid enthaltendes Lösungsmittel (Methylcellosolve oder Testbenzin) gegeben.

Die Tränkung der Probehölzer erfolgte durch 15minütiges Vakuum und folgender 18stündiger Druckbehandlung (2bar).

Die über 3 Tage getrockneten Hölzer wurden danach auf Malzextrakt-Agar, auf dem Coniophora puteana schon angewachsen war, gelegt. Die Inkubation der Probehölzer wurde für 12 Wochen bei 22°C und 75 % rel. Feuchte durchgeführt.

Ausgewertet wurde der Versuch visuell nach folgendem Schema:

- – = kein Bewuchs auf dem Holz, evtl. mit Hemmhof
- (-) = kaum sichtbarer, vereinzelter Bewuchs
- (+) = Holzstückchen teilweise bewachsen
- \+ = Holzstückchen stark bewachsen
- ++ = vollständig bewachsenes Holz

| Verb. Nr. | Einsatzkonz. % | Lösungsmittel | Bewuchs nach 9 Wo | Bewuchs nach 12 Wo |
|---|---|---|---|---|
| 1.22 | 0,1 | Testbenzin | - | - |
| 1.49 | 0,1 | Methylcellosolve | (-) | (-) |
| 1.49 | 0,5 | " | - | - |
| 1.50 | 0,1 | " | (+) | + |
| 1.50 | 0,5 | " | (-) | (+) |
| Wuchskontrolle | - | Testbenzin | (+) | ++ |
| " | - | Methylcellosolve | + | ++ |
| " | - | - | + | ++ |

Wie aus der Tabelle zu erkennen, können die Verbindungen auch im Holzschutz eingesetzt werden.

Patentansprüche

1. Verwendung der Verbindungen der Formel I

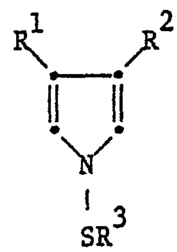

(I) ,

worin $R^1$ unsubstituiertes oder substituiertes Phenyl, Biphenyl, Pyridyl, Furyl oder Thienyl bedeutet, $R^2$ für $-COOR^4$, $-C(O)R^4$, $-CON(R^4)_2$, -CN, $-NO_2$, $-SO-R^4$, $-SO_2-R^4$, $-P(O)-(R^5)_2$ oder für $-SO_2-N(R^6)_2$ steht, wobei $R^4$ $C_1$-$C_6$ Alkyl, $R^5$ $C_1$-$C_6$ Alkoxy und $R^6$ $C_1$-$C_3$ Alkyl bedeutet und $R^3$ $C_1$-$C_3$ Halogenalkyl ist, als Biocide für den Materialschutz.

2. Verwendung gemäss Anspruch 1, wobei in der Formel I $R^1$ unsubstituiertes oder mit mindestens einem der Reste $R^7$, $R^8$ und/oder $R^9$ substituiertes Phenyl oder Biphenyl ist, und $R^1$ ferner unsubstituiertes oder mit mindestens einem der Reste $R^{10}$, $R^{11}$ und $R^{12}$ substituiertes Pyridyl, Furyl oder Thienyl bedeutet, wobei $R^7$, $R^8$ und $R^9$ unabhängig voneinander für Halogen, $C_1$-$C_4$ Alkyl, $C_1$-$C_3$ Halogenalkyl, $(R^4)_2N-$, $-NO_2$, -CN, $-COOR^4$ oder $-CON(R^4)_2$ steht oder eine der Gruppen $-OR^{13}$, $-SR^{13}$, $S(O)R^{13}$ oder $-SO_2-R^{13}$ ist, wobei $R^{13}$ $C_1$-$C_6$ Alkyl, $C_2$-$C_8$ Alkoxyalkyl, $C_3$-$C_5$ Alkenyl, $C_3$-$C_5$ Halogenalkenyl, $C_3$-$C_5$ Alkinyl, $C_3$-$C_5$ Halogenalkinyl, $C_3$-$C_5$ Hydroxyalkinyl, Phenyl oder Benzyl bedeutet, und $R^{10}$, $R^{11}$ und $R^{12}$ unabhängig voneinander Halogen oder $C_1$-$C_4$ Alkyl bedeuten, und $R^{10}$ zudem $-NO_2$ bedeutet und $R^2$, $R^3$, $R^4$, $R^5$ und $R^6$ die im Anspruch 1 angegebenen Bedeutungen haben.

3. Verwendung gemäss Anspruch 2, wobei $R^7$, $R^8$ und $R^9$ Fluor, Chlor, Brom, Methyl, Aethyl, Trifluormethyl, $(R^4)_2N-$, $-COOR^4$, $-CON(R^4)_2$ sind, oder eine Gruppe $-OR^{13}$ oder $-SR^{14}$ bedeuten, und $R^4$ Methyl oder Aethyl ist, und $R^{13}$ $C_1$-$C_4$ Alkyl, $C_3$-$C_5$ Alkenyl, $C_3$-$C_5$ Halogenalkyl,

Phenyl oder Benzyl bedeutet und $R^{14}$ $C_1$-$C_4$ Alkyl ist, und $R^{10}$, $R^{11}$ und $R^{12}$ Chlor, Brom, Methyl oder Aethyl sind, und $R^2$ $-COOR^6$, $-C(O)R^6$, $-CON(R^6)_2$, $-CN$, $-NO_2$, $-SO_2R^6$, $-P(O)(R^5)_2$ oder $-SO_2(NR^6)_2$ ist, wobei $R^5$ Methoxy oder Aethoxy ist und $R^6$ Methyl oder Aethyl ist, und $R^3$ $C_1$-$C_2$ Halogenalkyl bedeutet.

4. Verwendung gemäss Anspruch 2, wobei $R^1$ Phenyl, Pyridyl oder Furyl ist, und $R^7$, $R^8$ und $R^9$ Chlor, Brom, Methyl, Aethyl, Trifluormethyl, $-N(CH_3)_2$, $-COOCH_3$, $-CON(CH_3)_2$, $-OR^{13}$ oder $-SR^{14}$ ist, wobei $R^{13}$ $C_1$-$C_4$ Alkyl, Allyl, Phenyl oder Benzyl ist, und $R^{14}$ $C_1$-$C_4$ Alkyl bedeutet, und $R^{10}$, $R^{11}$ und $R^{12}$ Chlor, Brom oder Methyl ist, $R^2$ die im Anspruch 3 angegebene Bedeutung hat, wobei $R^5$ Aethoxy und $R^6$ Methyl ist, und $R^3$ $-CCl_3$, $-CCl_2F$, $-CCl_2H$, $-CF^3$, $-CF_2H$, $-C_2Cl_5$ oder $-CCl_2-CHCl_2$ ist.

5. Verwendung gemäss Anspruch 1, wobei $R^2$ $-C(O)CH_3$ oder $-CN$ ist.

6. Verwendung gemäss Anspruch 2, wobei $R^1$ Phenyl ist, $R^2$ $-C(O)CH_3$-CN oder $-SO_2CH_3$ bedeutet und $R^3$ $-CCl_3$ oder $-CCl_2F$ ist.

7. Verwendung gemäss Anspruch 1, wobei $R^1$ Phenyl, Pyridyl oder Furyl ist.

8. Verwendung gemäss Anspruch 7, wobei $R^1$ Phenyl ist.

9. Verwendung gemäss Anspruch 1, wobei $R^3$ $-CCl_3$, $-CCl_2F$ oder $-CCl_2CHCl_2$ ist.

10. Verwendung gemäss Anspruch 9, wobei $R^3$ $-CCl_2F$ ist.

11. Verwendung gemäss Anspruch 1 in Holzschutzmitteln.

12. Verwendung gemäss Anspruch 1 für die Wasserbehandlung.

13. Verwendung gemäss Anspruch 1 als Antischleimmittel in der Papierindustrie.

14. Verwendung gemäss Anspruch 1 in PVC-Weichfolien.

15. Verwendung gemäss Anspruch 1 in Dispersionsfarben und Antifouling-Farben.

16. Verwendung gemäss Anspruch 1 in Bohr- und Schneidölen.

17. Materialschutzmittel enthaltend als Biocid mindestens eine Verbindung der Formel I gemäss Anspruch 1.

Europäisches Patentamt

# EUROPÄISCHER RECHERCHENBERICHT

0111452

Nummer der Anmeldung

EP 83 81 0569

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 636 076 (SCHÜLKE & MAYR)<br>* Seiten 3-7 *<br>--- | 1-17 | A 01 N 43/36<br>A 01 N 43/40<br>A 01 N 47/04<br>A 01 N 57/24 |
| E | EP-A-0 096 142 (CIBA-GEIGY)<br>* Ansprüche 9-10 *<br>----- | 17 | |
| | | | RECHERCHIERTE SACHGEBIETE (Int. Cl. 3) |
| | | | A 01 N |

Der vorliegende Recherchenbericht wurde für alle Patentansprüche erstellt.

| Recherchenort | Abschlußdatum der Recherche | Prüfer |
|---|---|---|
| DEN HAAG | 20-03-1984 | DECORTE D. |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503. 03.82